# EUROPEAN PATENT APPLICATION

(11) **EP 1 576 892 A1**
(43) Date of publication of application: **21.09.2005**
(21) Application number: 03709842.3
(22) Date of filing: 07.04.2003
(51) Int. Cl.: A23L 1/29, A61K 31/70, A61K 35/78

(54) **METHOD FOR OBTAINING A PHYSIOLOGICAL TANNIN EXTRACT FROM BANANA TREES AND THE USE THEREOF AS FOOD COMPLEMENT**

(30) Priority: 16.04.2002 ES 200200894
(71) Applicant: Ruiz Gabaldon, Angel, 28028 Madrid (ES)
(72) Inventor: Ruiz Gabaldon, Angel, 28028 Madrid (ES)
(74) Representative: Garcia-Cabrerizo y del Santo, Pedro Maria
(86) International application number: PCT/ES2003/000158
(87) International publication number: WO 2003/086107

(57) **Abstract**

The invention relates to a method for obtaining a physiological tannin extract from banana trees stems. The method involves cooking the plants in hydroalcoholic water at a temperature of 30°-40° C during a 4-5 hour period. The resulting fluid extract is atomized by exposing said fluid to a hot air stream at a temperature of 70°-80° C during a fraction of a second. All the properties of the plant are thus preserved for use as food complement for human beings.

## Description

### OBJECT OF THE INVENTION

The present invention refers to the physiological tannin existing in the sap of plants formed in normal plant life conditions. Banana tree *(Musa spp.)* tannin object of this invention is contained at a high concentration in its sap, and its absorption is incorporated to the blood through the intestinal route as well as through the skin route.

### BACKGROUND OF THE INVENTION

Pathological tannins existing in plant trunk and branch galls, such as in holm oak, oak, elm, willow, fir, quebracho, and chestnut trees, etc., are used as tanners since the combination of tannin with hide gelatin forms a substance converting the hide into leather - rot-proof hide. Pathological tannins contain glycosides (bitter poisons against insects).

In contrast to these pathological tannins there are physiological tannins, found naturally in plants. The sap of banana tree leave contains 90% physiological tannins. Tannin is an astringent substance and the opposite, it hardens tissues and is eliminated through urine.

All physiological tannins naturally contained in plants are beneficial, regardless of a higher or lower concentration thereof. Any plant could be used, for example, such as the artichoke, apple tree or the leaves from the strawberry tree; the leaves from the banana tree are used in this case due to their high concentration of physiological tannin.

### DESCRIPTION OF THE INVENTION

The banana tree is a food plant yielding bananas *(Musa paradisiaca, Musa sapientum).*

This plant grows quickly, and the spike, which becomes the stem of the bunch, sprouts after two months, and the fruit is collected seven to twelve months later.

Each banana tree bears fruit only once, and once the fruit cluster is collected, the farmers cut the tree at the base in order to grow a new banana tree, and so on and so forth for fifty years.

The banana tree is not a tree, but rather a giant stem. The sap contained in its inner cells is very rich in physiological tannin, containing 90%. Once the banana cluster has been yielded, it can be used to extract its tannin, sending it to plant laboratories.
1. The trunk leaves are washed well and those that are not optimal are discarded.
2. Once the plant has been well washed with water, it is cut and ground in order to obtain the extract thereof by means of atomization.

### DETAILED DESCRIPTION OF THE INVENTION

The banana tree is a food plant of vegetable-origin. If the most up-to-date technology is used to obtain the extract thereof, that is atomization, preservation of each and every one of the original plant characteristics can be assured.

The atomization process consist of boiling the plant at a temperature of 30-40°C in hydroalcoholic water (ethanol) for a time of four to five hours, and the fluid extract will be nebulized, passing a hot air current (+70-80°C) through it for a fraction of a second. Immediate evaporation of the solvent (ethanol) occurs and the dust collected in particles of one micrometer in diameter is cooled. The collected extract preserves the essential oils and active ingredients of the plant.

With nebulization, a dry extract is obtained by atomization in which technical treatments of the plant are so low that the essential oil contents of the plant are not lost.

The wastes (fibers) resulting from atomizing the banana trees are applicable as a plant layer for farms and as fertilizer.

### APPLICATION OF THE EXTRACT

First and foremost, physiological tannin is a natural food suitable for human consumption. It favors good digestion and assimilation of foods.

The dry extract taken as a food complement is the best stabilizer for the human organism, so that foreign agents cannot be introduced nor cells of the organism become degenerated.

The dose for healthy people is to ingest 1 gram of tannin dissolved in 200 mL of water (1 glass) a day in order to prevent destabilizing states.

The dose for sick people is to increase the tannin up to 5 grams dissolved in one liter of water a day. In both cases, the tannin will be taken when freshly diluted.

The obtained extract can be mixed with honey or jelly in order to obtain an ideal candy for smokers.

## Claims

1. A method for obtaining a tannin extract from banana trees, **characterized in that** once the banana cluster is cut, the stems are washed, cut up, and boiled for four to five hours at a temperature of 30-40° C; the steeped liquid resulting from boiling is nebulized, atomizing it so that a dry extract is left.

2. A process according to claim 1, **characterized in that** the steeped liquid resulting from boiling is nebulized, atomizing it so that a dry extract is left in particles of one micrometer in diameter.

3. The extract obtained by means of the procedure of claims 1 and 2 mixed with honey or royal jelly is an ideal candy for smokers.

4. Use of the extract obtained according to claims 1 to 3 as a food complement for humans.
